(19) Europäisches Patentamt
European Patent Office
Office européen des brevets

(11) **EP 4 484 453 A1**

(12) **EUROPEAN PATENT APPLICATION**
published in accordance with Art. 153(4) EPC

(43) Date of publication:
**01.01.2025  Bulletin 2025/01**

(21) Application number: 23760336.0

(22) Date of filing: **17.02.2023**

(51) International Patent Classification (IPC):
*C08B 37/08* (2006.01)    *A61K 9/00* (2006.01)
*A61K 47/36* (2006.01)    *A61P 27/02* (2006.01)
*A61P 27/04* (2006.01)    *A61K 9/08* (2006.01)

(86) International application number:
**PCT/KR2023/002353**

(87) International publication number:
**WO 2023/163462 (31.08.2023 Gazette 2023/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.02.2022  KR 20220023811**

(71) Applicant: Scai Therapeutics Co., Ltd.
**Seoul 06524 (KR)**

(72) Inventors:
• KIM, Chul Hwan
  **Daejeon 35246 (KR)**
• KIM, Kyoung Hee
  **Daejeon 35246 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **HIGH-MOLECULAR-WEIGHT HYALURONIC ACID STRUCTURE, METHOD FOR PREPARING SAME, AND OPHTHALMIC COMPOSITION INCLUDING SAME**

(57)    The present invention relates to a high-molecular-weight hyaluronic acid structure obtained by applying shear stress to a solution containing high-molecular-weight hyaluronic acid, a method for preparing the same, and an ophthalmic composition including the same.

【Figure 3】

50 nm

EP 4 484 453 A1

**Description**

[Technical Field]

This application claims the benefit of priority based on Korean Patent Application No. 10-2022-0023811 filed on February 23, 2022, the entire contents thereof are included as part of this specification.
The present invention relates to a high-molecular-weight hyaluronic acid structure, a method for preparing the same, and an ophthalmic composition including the same, and more specifically, to a high-molecular-weight hyaluronic acid structure with reduced viscosity by applying shear stress to high-molecular-weight hyaluronic acid, a method for preparing the same, and an ophthalmic composition including the same.

[Background Art]

Dry eye syndrome refers to a condition in which the eye is dry due to a malfunction of the lacrimal gland, resulting in low tear production or excessive evaporation of tears. This is one of the diseases that is accompanied by increased osmotic pressure and inflammation of the ocular surface due to drying of the tear film, leading to discomfort in daily life and even blindness.
Eye drops are the most used to relieve and treat dry eye syndrome. Commercially available eye drops were developed mainly to focus on moisture reinforcement through replenishment of the aqueous layer, the middle layer of tears, and serve to prevent moisture evaporation through polymer substances. These polymer substances include hyaluronic acid, carboxymethyl cellulose (CMC), hydropropylmethyl cellulose (HPMC), and polyvinyl alcohol (PVA) .
Among these polymer substances, hyaluronic acid is a biopolymer present in the human body, which is widely distributed in the skin, vitreous body of the eye, joint fluid, muscle, umbilical cord, cockscomb, etc., and exerts various functions in each part of the body. In addition, hyaluronic acid is very soluble in water, making it easy to develop into eye drops, so hyaluronic acid eye drops account for about 70% of the artificial tear market.
Eye drops marketed with hyaluronic acid as the main ingredient contain hyaluronic acid in concentrations of 0.1%, 0.15%, 0.18%, and 0.3%, and low-concentration products are generally sold.
Even if the concentration of hyaluronic acid is the same, the viscosity varies depending on the molecular weight. The higher the molecular weight of hyaluronic acid, the higher the viscosity. In the case of existing eye drops containing hyaluronic acid, the higher the viscosity of the eye drops, the more effective it is in relieving symptoms. However, they can irritate the eyes of patients with dry eye syndrome, cause a sticky or stiff feeling when blinking, and cause blurred vision.
There is an increasing demand for eye drops that control viscosity while adding hyaluronic acid at a high concentration or containing high molecular weight hyaluronic acid at the same concentration.

[Prior Art Documents]

[Patent Document]

(Patent Document 1) Korean Laid-open Patent Publication No. 10-2021-0015377 (2021.02.10), A method for preparing hyaluronic acid with low viscosity

[Disclosure]

[Technical Problem]

The technical problem to be achieved by the present invention is to provide a high-molecular-weight hyaluronic acid structure, a method for preparing the same, and an ophthalmic composition including the same. More specifically, it is to provide a high-molecular-weight hyaluronic acid structure with reduced viscosity without reducing the molecular weight by applying shear stress to the high-molecular-weight hyaluronic acid, a method for preparing the same, and an ophthalmic composition including the same.
The technical problem to be achieved by the present invention is not limited to the mentioned technical problem, and other technical problems not mentioned will be clearly understood by those skilled in the art from the description below.

[Technical Solution]

In order to achieve the above technical problem, the viscosity of a solution containing a high-molecular-weight hyaluronic acid structure according to one aspect of the present invention may be 1 to 60 cP.
In an embodiment of the present invention, the molecular weight of the hyaluronic acid may be in the range of $5.0 \times$

$10^5$ to $2.0 \times 10^6$ g/mol.

In an embodiment of the present invention, the solution containing the structure may contain hyaluronic acid content in the range of 0.01 %(w/v) to 0.5 %(w/v).

In an embodiment of the present invention, when the molecular weight and concentration of the structure and hyaluronic acid, a precursor of the structure, are the same, a/b, which is the ratio of the viscosity (a) of the solution containing the structure and the viscosity (b) of the solution containing hyaluronic acid, a precursor of the structure, may be 0.001 to 0.30.

In an embodiment of the present invention, the intrinsic viscosity of the solution containing the structure may be 0.1 to 2.5 $m^3$/kg.

The present invention can provide an ophthalmic composition containing the high-molecular-weight hyaluronic acid structure.

In an embodiment of the present invention, the viscosity of the ophthalmic composition may be 1.0 to 30.0 cP.

In an embodiment of the present invention, the ophthalmic composition may further comprise excipients.

In an embodiment of the present invention, the excipients may be at least one selected from the group consisting of buffering agents, preservatives, tonicity regulators, and pH regulators.

The present invention can provide an ophthalmic composition for prevention or treatment of dry eye syndrome, including the above-described ophthalmic composition.

The present invention can provide a kit comprising a container containing the ophthalmic composition, wherein the container includes a dispensing means suitable for topical administration of the ophthalmic composition.

In an embodiment of the present invention, the dispensing means provides the ophthalmic composition dropwise in droplets having a volume of 0.01 to 0.10 ml.

[Advantageous Effects]

The present invention, which manufactures a new structure of hyaluronic acid using intramolecular interactions such as ionic bonds such as hydrogen bonds and/or interactions such as ionic clusters while maintaining the same high molecular weight, has the advantage of significantly lowering viscosity and improving feeling of use.

In addition, the ophthalmic composition containing the high-molecular-weight hyaluronic acid structure of the present invention has the advantage of improving moisture retention due to the large amount of hyaluronic acid in the ophthalmic composition.

[Description of Drawings]

FIG. 1 is a diagram showing the polymer chain of hyaluronic acid, the precursor of the structure according to an embodiment of the present invention.

FIG. 2 is a diagram showing a single polymer chain of hyaluronic acid, the precursor of the structure according to an embodiment of the present invention.

FIG. 3 is a Scanning Electron Microscope (SEM) image of an aqueous solution containing the structure according to an embodiment of the present invention.

FIG. 4 is a Scanning Electron Microscope (SEM) image of an aqueous solution containing the structure according to an embodiment of the present invention.

FIG. 5 is a Transmission Electron Microscope (TEM) image of hyaluronic acid, the precursor of the structure according to an embodiment of the present invention.

FIG. 6 shows a comparison of HPLC measurement results according to an embodiment of the present invention.

[Best Mode]

In general, unlike the structure of molecules with low molecular weight, polymers have a long chain structure with regular repeating units through chemical reactions of monomer molecules. The structure of polymers with long chains exists in a solid state or has a twisted chain structure when in a liquid state.

In very dilute polymer solutions, viscosity is related to volume. This is well known as Einstein's formula, and can be expressed as Equation 1 below:

[Equation 1]

$$\eta_{rel} = \frac{\eta}{\eta_0} = 1 + 2.5\phi + f(\phi)$$

(wherein, $\eta$ is the viscosity of the solution, $\eta_0$ is the viscosity of the solvent, $\eta_{rel}$ is the relative viscosity, and $\phi$ represents the volume).

The larger the molecular weight, the greater the affinity between the solvent and the polymer, and the greater the repulsive force between unit chain structures, the larger the volume per unit mass of the polymer solution.

In addition, as the concentration of the polymer solution increases, the chains penetrate into each other to form a very large continuum structure, and as the polymer concentration becomes diluted, the interaction between each chain decreases.

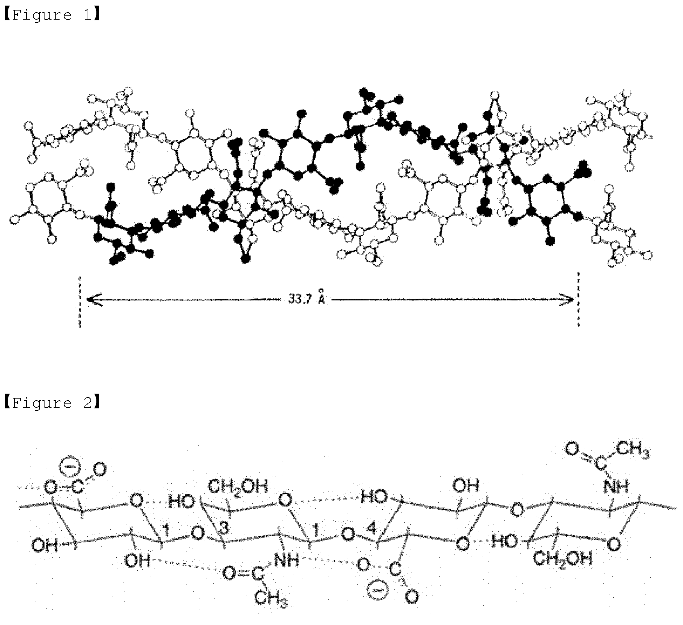

Unlike ionic polymers in which each chain exists independently, hyaluronic acid polymers are known to form a twin helix structure in which two chains are bundled together in aqueous solution. This is also called twin helix or macromolecular crowding. FIG. 1 shows a schematic diagram in which two chains of a hyaluronic acid polymer are entangled with each other to form a twin helix structure.

In general, an ophthalmic composition containing high molecular weight hyaluronic acid promotes collagen synthesis and has an excellent moisture retention effect due to its long residence time in the eye compared to eye drops containing low molecular weight hyaluronic acid. However, when applying high molecular weight hyaluronic acid to the eye and blinking, there is a problem in that the stickiness is high and the feeling of use is poor. To solve these problems, many studies are being conducted to control the viscosity of polymer solutions.

However, these methods for controlling viscosity had limitations in that they did not maintain the characteristics of the polymer of hyaluronic acid, such as lowering the viscosity of the polymer solution by reducing the molecular weight by causing a hydrolysis reaction of the polymer chain of hyaluronic acid through pH adjustment and addition of catalyst.

In order to solve the above-mentioned problem, the present inventors converted a pair of polymer chains of high-molecular-weight hyaluronic acid with very high viscosity into a single helix structure by applying high shear stress, and confirmed that ionic associations are formed through intramolecular interactions and/or ionic cluster interactions between single polymer chains.

Through this, it was confirmed that the viscosity was significantly lowered because the volume of the high-molecular-weight hyaluronic acid chain was reduced and the volume of the high-molecular-weight hyaluronic acid in the solution was reduced.

In the following, a method for manufacturing a high-molecular-weight hyaluronic acid structure according to an embodiment of the present invention will first be described, and then the high-molecular-weight hyaluronic acid structure will be described in detail.

**Method for manufacturing high-molecular-weight hyaluronic acid structure**

The term "high-molecular-weight hyaluronic acid structure" used herein refers to a structure having a single polymer chain structure formed by applying shear stress to high-molecular-weight hyaluronic acid having a pair of polymer chain structures.

As used herein, the term "precursor" refers to a pro-drug or a leading compound used to produce the high-molecular-weight hyaluronic acid structure according to the present invention. In other words, the precursor of the high-molecular-weight hyaluronic acid structure according to the present invention refers to hyaluronic acid having a pair of polymer chain structure to which shear stress is not applied.

The high-molecular-weight hyaluronic acid structure according to an embodiment of the present invention can be manufactured by applying shear stress to a solution containing hyaluronic acid, a precursor of the structure.

The shear stress applied to the solution containing hyaluronic acid, the precursor of the structure, may be either mechanical shear stress or ultrasonic wave application.

The mechanical shear stress may be applied by dispersing a solution containing hyaluronic acid, the precursor of the structure, at high pressure by attaching it to the inlet of a high pressure disperser. In addition, mechanical shear stress can be applied by injecting a solution containing hyaluronic acid, the precursor of the structure, into a mill such as a ball mill, pin mill, or roll mill. Below, mechanical shear stress is explained in detail. When a solution containing hyaluronic acid, the precursor, passes through the nozzle of a high-pressure disperser at high speed, it passes through a physically narrow area, causing hyaluronic acid, the precursor of the structure, to undergo very high shear stress.

The shear stress may be applied using ultrasonic wave. Hereinafter, application of ultrasonic wave will be described in detail.

According to an embodiment of the present invention, the shear stress may be applied by applying ultrasonic wave to a solution containing the hyaluronic acid.

When the ultrasonic wave is applied to a solution containing the hyaluronic acid, a pressure wave is generated, and shear stress may be applied to hyaluronic acid, the precursor of the structure, by the pressure wave.

The intensity of the applied ultrasonic wave may be 200 J/sec to 800 J/sec or 400 J/sec to 600 J/sec.

The energy applied per volume of the applied ultrasonic wave can be calculated as Intensity of ultrasonic wave (J/sec) x Applied time (sec)/Measured volume (ml).

According to an embodiment of the present invention, the energy applied per volume of ultrasonic wave applied to the solution containing the hyaluronic acid may be 100 J/ml to 90 kJ/ml. If the energy of the ultrasonic wave is less than 100 J/ml, sufficient shear stress is not applied to the solution containing the hyaluronic acid, making it difficult to form a structure. Additionally, when the energy of the ultrasonic wave exceeds 90 kJ/ml, excessive heat is applied to the solution containing the hyaluronic acid, making it difficult to form a structure.

The ultrasonic wave can be applied at 10°C to 80°C for 10 seconds to 500 minutes. When the ultrasonic wave is applied at a temperature less than 10°C, there is no change in the solution containing the hyaluronic acid, and when applied at a temperature exceeding 80°C, a phase change occurs in the solution containing the hyaluronic acid, making it difficult to form the high-molecular-weight hyaluronic acid structure according to the present invention. In addition, when the ultrasonic wave is applied for less than 10 seconds, there is no change in the solution containing the hyaluronic acid, and when applied for a time exceeding 500 minutes, the structure of the solution containing the hyaluronic acid is deformed, so the high-molecular-weight hyaluronic acid structure according to the present invention cannot be formed.

## High-molecular-weight hyaluronic acid structure

The high-molecular-weight hyaluronic acid structure according to the present invention can be manufactured by the manufacturing method described above. Specifically, the high-molecular-weight hyaluronic acid structure can form a single polymer chain by applying shear stress to hyaluronic acid, the precursor of the structure.

The single polymer chain may have a structure containing the chemical structure of hyaluronic acid as shown in FIG. 2. The chemical structure of the hyaluronic acid may be a single polymer chain having one carboxyl anion ($-COO^-$) group for every two cyclic hexoses.

The single polymer chain may form an ionic aggregate due to intramolecular interactions and/or ionic cluster interactions as the distance between single chains becomes closer. The intramolecular interaction may be achieved through intramolecular hydrogen bonding, ionic bonding, etc. The interaction between the ionic clusters and the like can be achieved through interaction between partial charges of carboxyl anions ($-COO^{\delta-}$) and sodium cations ($Na^{\delta+}$) in hyaluronic acid sodium salt.

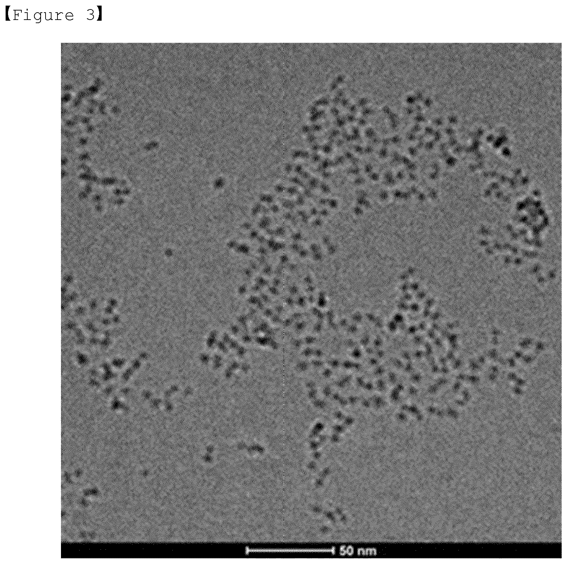

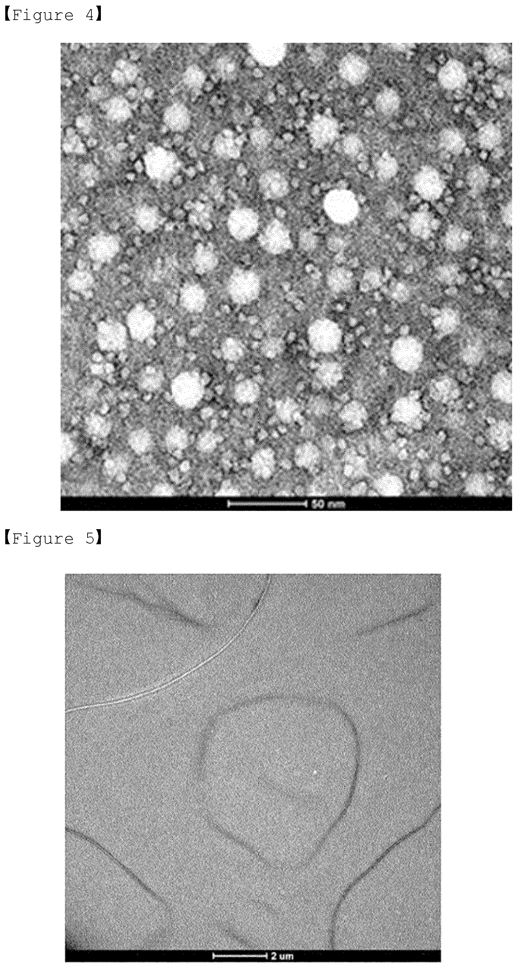

The ionic aggregate may include a spherical structure as shown in FIGS. 3 and 4. The ionic cluster may have a spherical structure with a diameter of 1 to 20 nm, 2 to 18 nm, or 3 to 16 nm due to the intramolecular interactions, ionic cluster interaction or the like.

On the other hand, hyaluronic acid, the precursor of the structure according to the present invention, has a general polymer chain structure and does not contain an ionic aggregate having the spherical structure. FIG. 5 is a TEM image of hyaluronic acid, the precursor of the structure according to the present invention, wherein lines were observed as marks created during the freezing process for TEM imaging of the hyaluronic acid, but the shape of the ionic aggregate with a spherical structure was not observed.

## Molecular weight of high-molecular-weight hyaluronic acid structure

Molecular weight of the high-molecular-weight hyaluronic acid structure according to the present invention may be $5.0 \times 10^5$ to $2.0 \times 10^6$ g/mol, $5.5 \times 10^5$ to $1.95 \times 10^6$ g/mol, or $6.0 \times 10^5$ to $1.90 \times 10^6$ g/mol.

The high-molecular-weight hyaluronic acid structure according to the present invention has the same molecular weight as hyaluronic acid, the precursor of the structure. That is, even if a high-molecular-weight hyaluronic acid structure is manufactured by applying shear stress to a solution containing hyaluronic acid, the precursor of the structure, it is possible to manufacture a structure that maintains the same molecular weight as hyaluronic acid, the precursor of the structure before applying shear stress, and has lower viscosity.

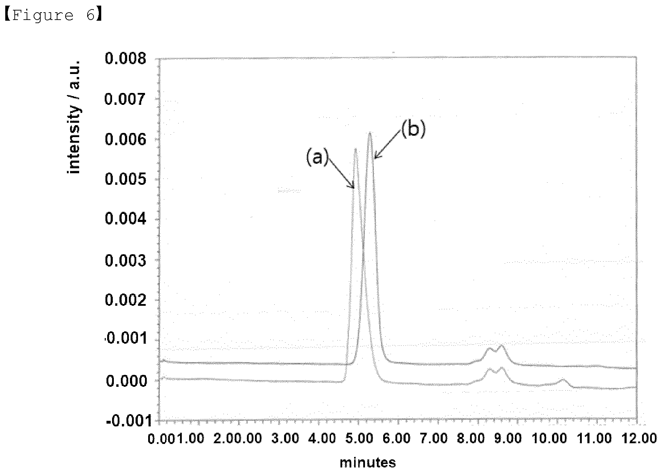

In FIG. 6, (a) shows the molecular weight measurement curve of hyaluronic acid sodium salt, which is the precursor of the structure according to the present invention, and (b) shows the molecular weight measurement curve of the high-molecular-weight hyaluronic acid structure according to the present invention.

As shown in FIG. 6, it was confirmed that the molecular weight measurement curve of hyaluronic acid, the precursor of the structure according to the present invention, and the molecular weight measurement curve of the high-molecular-weight hyaluronic acid structure according to the present invention were the same with almost no change. Therefore, the high-molecular-weight hyaluronic acid structure according to the present invention can provide a high-molecular-weight hyaluronic acid structure with lowered viscosity without a decrease in molecular weight even when shear stress is applied to hyaluronic acid, the precursor of the structure.

The high-molecular-weight hyaluronic acid structure according to the present invention can have the effect of improving moisture retention by lowering viscosity, eliminating stickiness when applied to the eye and improving the feeling of use, while maintaining the effects of high molecular weight hyaluronic acid, such as water retention and excellent epithelial regeneration ability.

**Viscosity of high-molecular-weight hyaluronic acid structure**

The viscosity of a solution containing a high-molecular-weight hyaluronic acid structure with a molecular weight of $5.0 \times 10^5$ to $2.0 \times 10^6$ g/mol and a concentration of 0.1 to 0.5% prepared according to an embodiment of the present invention may be 1 to 60 cP.

According to an embodiment of the present invention, when shear stress is applied to the solution containing a high-molecular-weight hyaluronic acid, the precursor of the high-molecular-weight hyaluronic acid structure, the physical structure of hyaluronic acid is transformed from a twin helix structure to a single polymer chain, lowering the viscosity while maintaining the high molecular weight of hyaluronic acid.

The reason why the viscosity of the solution containing a high-molecular-weight hyaluronic acid structure according to the present invention is significantly lowered is that by applying shear stress to the solution containing hyaluronic acid, the precursor of the structure, the single polymer chain is formed, and an ionic aggregate is formed within the single polymer chain, thereby reducing the volume of the polymer chain, and reducing the volume of the high-molecular-weight hyaluronic acid in the solution.

According to an embodiment of the present invention, when the molecular weight and concentration of the high-molecular-weight hyaluronic acid structure and hyaluronic acid, the precursor of the structure, are the same, a/b, which is the ratio of the viscosity (a) of the solution containing the structure and the viscosity (b) of the solution containing hyaluronic acid, the precursor of the structure, may be 0.001 to 0.30.

**Contact angle of high-molecular-weight hyaluronic acid structure**

The contact angle of the solution containing a high-molecular-weight hyaluronic acid structure prepared according to an embodiment of the present invention may be 20° to 30° on a hydrophilic glass surface and 80° to 100° on a hydrophobic glass surface.

The contact angle measurement method may include a sessile drop method for measuring a static contact angle, a tilting drop method, a captive drop method, and a wilhelmy plate method for measuring a dynamic contact angle, and preferably, the sessile drop method may be used.

The hydrophilic glass surface may be a glass surface containing silica material without any treatment.

The hydrophobic glass surface may be a glass surface containing a material coated with Teflon emulsion.

**Intrinsic viscosity of high-molecular-weight hyaluronic acid structure**

Intrinsic viscosity refers to the volume per unit mass of polymer when there is no viscous action of the polymer solution.

In the method of measuring the intrinsic viscosity, intrinsic viscosity can be measured by measuring the aqueous solution to be measured according to its concentration, calculating the reduced viscosity, and extrapolating this to read the reduced viscosity at concentration 0. The intrinsic viscosity can be calculated using Equation 4 below.

Equation 2 below is a formula for calculating relative viscosity, and Equation 3 below is a formula for calculating reduced viscosity (wherein, $\eta$ represents the viscosity of the solution, $\eta_0$ represents the viscosity of the solvent, and c represents the concentration of the solution). Generally, the concentration (c) of a solution is expressed in units of (w/v) because the physical meaning of $\eta_{red}$ is the volume per unit weight of the polymer in the solution. The reduced viscosity is graphed according to concentration, and the intercept obtained by linearly extrapolating the point where the concentration becomes 0, as described above, becomes the intrinsic viscosity.

$$[\text{Equation 2}]$$

$$\eta_{re} = \eta/\eta_0$$

(wherein, $\eta$ represents the viscosity of the solution and $\eta_0$ represents the viscosity of the solvent).

[Equation 3]

$$\eta_{red} = \left(\frac{\eta}{\eta_0} - 1\right)/c$$

(wherein, $\eta$ represents the viscosity of the solution, $\eta_0$ represents the viscosity of the solvent, and c represents the concentration of the solution).

[Equation 4]

$$[\eta] = \lim_{c \to \infty} \frac{(\eta_{re} - 1)}{c}$$

(wherein, $\eta_{red}$ represents the relative viscosity and c represents the concentration of the solution).

The intrinsic viscosity of the solution containing the high-molecular-weight hyaluronic acid structure according to the present invention can be calculated based on Equations 2 to 4 above. The specific intrinsic viscosity measurements of the solution containing the high-molecular-weight hyaluronic acid structure according to the present invention are shown in Experimental Example 8 below.

**Ophthalmic composition containing high-molecular-weight hyaluronic acid structure**

According to an embodiment of the present invention, an ophthalmic composition containing the high-molecular-weight hyaluronic acid structure can be provided.

The ophthalmic composition may include the characteristics of the high-molecular-weight hyaluronic acid structure according to the present invention as it is.

The description of the high-molecular-weight hyaluronic acid structure is the same as described above.

Additionally, the ophthalmic composition according to the present invention may contain other conventional ingredients such as one or more pharmaceutically acceptable buffering agents, preservatives, tonicity regulators, pH adjusters, etc.

The pH of the ophthalmic composition according to an embodiment of the present invention is preferably 5.5 as a lower limit, more preferably 6, more preferably exceeding 6, and preferably 8 as an upper limit, more preferably 7.5, and even more preferably 7. Within this pH range, hyaluronic acid or its salt in the ophthalmic composition can be stabilized and can be suitably used as an ophthalmic composition as it is hypoallergenic.

The ophthalmic composition according to an embodiment of the present invention may further include a buffering agent suitable for maintaining the above-mentioned preferred pH range. Examples of the buffering agent include, but are not limited to, bicarbonate buffer, acetate buffer, citrate buffer, phosphate buffer, borate buffer, or tromethamine (TRIS, 2-amino2-hydroxymethyl-1,3-propanediol) buffer, and combinations thereof. The amount (concentration) of the buffering agent added is not particularly limited, as long as the pH of the ophthalmic composition according to an embodiment of the present invention can be maintained in the above-mentioned preferred range.

The ophthalmic composition according to an embodiment of the present invention may further include a preservative suitable for preventing microbial contamination. The preservative may include any compound or substance. The preservative may be selected from the group consisting of: persalts such as perborate and percarbonate; alcohols such as benzyl alcohol and chlorobutanol; preservatives containing quaternary ammonium salts such as benzalkonium chloride, benzalkonium bromide, and polyquaternium; guanidine-based preservatives including polyhexamethylene biguanidine (PHMB) and chlorhexidine; mercury preservatives such as thimerosal, phenylmercuric acetate, and phenylmercuric nitrate; metal chlorites such as alkali metal and alkaline earth metal chlorites; ophthalmologically acceptable salts such as sorbic acid and potassium sorbate and mixtures thereof; and oxidizing preservatives such as stabilized oxychloro complexes (e.g., Purite® (registered trademark of Allergan, Inc.)). The amount of the preservative varies over a relatively wide range depending on the specific preservative used. If the preservative is not added to the ophthalmic composition, the ophthalmic composition can be used as a disposable eye drop, and the ophthalmic composition is consumed in one administration. Alternatively, the ophthalmic composition may be used, for example, as a multiple-use eye drop contained in a container with a filter attached to the nozzle of the container for dispensing the eye drop, or contained in an airless application system device.

The ophthalmic composition according to an embodiment of the present invention may further include a tonicity regulator that adjusts the osmotic pressure of the eye drops to be similar to the osmotic pressure within the eye to eliminate irritation and pain due to the difference in osmotic pressure when instilling the eye drops. The tonicity regulator may be in ionic and/or nonionic form. The ionic tonicity regulator is, for example, alkali metal or earth metal halides, one or more of the following: calcium chloride, potassium chloride, sodium chloride, lithium chloride, potassium bromide, sodium bromide, sodium iodide, sodium phosphate, potassium phosphate, sodium and potassium sulfate, sodium and potassium bicarbonate, and boric acid. The nonionic tonicity regulator is, for example, urea, glycerol, sorbitol, mannitol, propylene glycol, dextrose, or a combination thereof. Glycerin, sodium chloride and mannitol are the most preferred tonicity regulators. The amount of the tonicity regulator may vary depending on whether an isotonic, hypertonic or hypotonic liquid is required. The composition of the present invention generally has an osmotic pressure in the range of 150-1500 mOsm/kg, preferably 150-500 mOsm/kg, and most preferably 180-250 mOsm/kg.

The ophthalmic composition according to an embodiment may further contain a pH adjuster in order to adjust the pH to the above-mentioned preferable range. The pH adjusting agent is not particularly limited as long as it can adjust the pH of the ophthalmic composition according to an embodiment of the present invention, and specific examples include diluted hydrochloric acid, sodium hydroxide, etc. As long as the pH of the ophthalmic composition according to an embodiment of the present invention can be adjusted to the above desirable range, the amount (concentration) of the pH adjusting agent added is not particularly limited.

The ophthalmic composition according to an embodiment of the present invention can be prepared by dissolving the components in an aqueous medium. Deionized water is a preferred aqueous medium that may contain small amounts of other hydrophilic solvents such as glycols and/or polyols. A composition can be prepared by preparing a solution of one or more ingredients and then adding the remaining one or more ingredients, or by preparing two or more separate solutions, each containing one or more ingredients, and then mixing these solutions together.

The ophthalmic composition according to an embodiment of the present invention may be an ophthalmic composition for prevention or treatment of dry eye syndrome.

The present invention provides a kit comprising a container including the ophthalmic composition. The container including the ophthalmic composition comprises a dispensing means suitable for topical administration of the ophthalmic composition to the eye of a patient. The dispensing means can provide the ophthalmic composition dropwise in droplets having a volume of 0.01 to 0.10 ml, but is not limited to the volume and includes the volume per drop typically applied to the eye.

Hereinafter, examples of the present invention will be described in detail so that those skilled in the art can easily practice the present invention. However, the present invention may be implemented in various different forms and is not limited to the Example described herein.

**Preparation of high-molecular-weight hyaluronic acid structure**

**[Example 1]**

Hyaluronic acid sodium salt (sodium hyaluronate, a product from Shiseido company, Japan) (0.5 g) with a molecular weight of $9.0 \times 10^5$ g/mol was placed in a mass cylinder, and distilled water was added so that the total volume was 100 ml. At 25°C, the solution was stirred until it became uniform, thereby preparing an aqueous solution of hyaluronic acid sodium salt with a concentration of 0.5% (w/v).

The prepared aqueous solution of hyaluronic acid sodium salt was put into the inlet of a high-pressure disperser (NLM 100 from Ilshin Autoclave) and dispersed under high pressure. After being stabilized at room temperature for 1 hour, an aqueous solution containing a high-molecular-weight hyaluronic acid structure was prepared.

**[Example 2]**

An aqueous solution containing a high-molecular-weight hyaluronic acid structure was prepared in the same manner as in Example 1, except that the aqueous solution of hyaluronic acid sodium salt prepared in Example 1 was prepared by repeating high-pressure dispersion through a high-pressure disperser two and three times.

**[Example 3]**

An aqueous solution containing a high-molecular-weight hyaluronic acid structure was prepared in the same manner as in Example 1, except that hyaluronic acid sodium salt (sodium hyaluronate, a product from Shiseido company, Japan) (0.5 g) with a molecular weight of $1.2 \times 10^6$ g/mol was used.

**[Example 4]**

An aqueous solution containing a high-molecular-weight hyaluronic acid structure was prepared in the same manner as in Example 2, except that the aqueous solution of hyaluronic acid sodium salt prepared in Example 3 was prepared by repeating high-pressure dispersion through a high-pressure disperser two, three and four times.

**[Example 5]**

Hyaluronic acid sodium salt (sodium hyaluronate, a product from Shiseido company, Japan) (0.3 g) with a molecular weight of $9.0 \times 10^5$ g/mol was placed in a mass cylinder, and distilled water was added so that the total volume was 100 ml. At 25°C, the solution was stirred until it became uniform, thereby preparing an aqueous solution of hyaluronic acid sodium salt with a concentration of 0.3% (w/v).

Using an ultrasonic wave homogenizer (ultrasonic homogenizer, HD4200 from BANDELIN company, Germany), an ultrasonic wave was applied to the prepared aqueous solution of hyaluronic acid sodium salt at 25°C for about 20 minutes to prepare an aqueous solution containing a high-molecular-weight hyaluronic acid structure. The total energy of the applied ultrasonic wave varies depending on time, and an energy of about 1.5 kJ to 2.5 kJ was applied per minute.

**[Comparative Example 1]**

Hyaluronic acid sodium salt (sodium hyaluronate, a product from Shiseido company, Japan) (0.5 g) with a molecular weight of $9.0 \times 10^5$ g/mol was placed in a mass cylinder, and distilled water was added so that the total volume was 100 ml. At 25°C, the solution was stirred until it became uniform, thereby preparing an aqueous solution of hyaluronic acid sodium salt with a concentration of 0.5% (w/v).

**[Comparative Example 2]**

Hyaluronic acid sodium salt (sodium hyaluronate, a product from Shiseido company, Japan) (0.5 g) with a molecular weight of $1.2 \times 10^6$ g/mol was placed in a mass cylinder, and distilled water was added so that the total volume was 100 ml. At 25°C, the solution was stirred until it became uniform, thereby preparing an aqueous solution of hyaluronic acid sodium salt with a concentration of 0.5% (w/v).

**Experimental Example 1: Comparison of viscosity of Examples 1 to 4 and Comparative Examples 1 and 2**

The viscosity of the aqueous solutions of Examples 1 to 4 and Comparative Examples 1 and 2 was measured at 25°C using a viscosity meter (Brookfield company's DV2T viscometer). The measured viscosity is listed in Table 1 and Table 2 below. Aqueous solutions prepared by repeating high pressure dispersion through a high pressure disperser two and three times in Example 2 were designated as Example 2-1 and Example 2-2, respectively. In addition, aqueous solutions prepared by repeating high pressure dispersion through a high pressure disperser two, three, and four times in Example 4 were designated as Examples 4-1, 4-2, and 4-3, respectively.

[Table 1]

|  | Comparative Example 1 | Example 1 | Example 2-1 | Example 2-2 |
|---|---|---|---|---|
| **Viscosity (cP)** | 670±55 | 22±3 | 10±1 | 10±0.9 |

[Table 2]

|  | Comparative Example 2 | Example 3 | Example 4-1 | Example 4-2 | Example 4-3 |
|---|---|---|---|---|---|
| **Viscosity (cP)** | 1,150±90 | 31±2 | 15±2 | 10±1 | 10±0.8 |

From the results in Table 1 above, it was confirmed that the viscosity of the aqueous solution containing the high-molecular-weight hyaluronic acid structure prepared in Examples 1 and 2 is significantly lower than the viscosity of the hyaluronic acid sodium salt solution, which is the precursor of the structure, prepared in Comparative Example 1. In addition, it could be confirmed that as high pressure dispersion was applied repeatedly several times, the viscosity decreased to about 10 cP.

From the results in Table 2 above, it was confirmed that the viscosity of the aqueous solution containing the high-molecular-weight hyaluronic acid structure prepared in Examples 3 and 4 is significantly lower than the viscosity of the hyaluronic acid sodium salt solution, which is the precursor of the structure, prepared in Comparative Example 2. In addition, it could be confirmed that as high pressure dispersion was applied repeatedly several times, the viscosity decreased to about 10 cP.

Through this, It was found that when a specific shear stress is applied to the sodium salt of hyaluronic acid, which is a precursor, a twin helix structure of hyaluronic acid is converted into a single helix structure, and a high-molecular-weight hyaluronic acid structure in which ionic aggregates are formed within the single polymer chain is manufactured. The reason why the viscosity of the solution containing the high-molecular-weight hyaluronic acid structure according to the present invention is significantly lowered is because the ionic cluster is formed within the single polymer chain, which reduces the volume of the polymer chain, thereby reducing the volume of the high-molecular-weight hyaluronic acid in the solution.

Therefore, even if it contains high molecular weight hyaluronic acid, a solution with lowered viscosity can be prepared without changing the molecular weight.

**Experimental Example 2: Viscosity change according to ultrasonic wave application time**

The viscosity of the aqueous solution containing the high-molecular-weight hyaluronic acid structure prepared in Example 5 was measured. The change in viscosity of the aqueous solution according to the ultrasonic wave application time is shown in Table 3 below.

[Table 3]

| Ultrasonic wave application time(min) | Viscosity (cP) | Ratio before and after ultrasonic wave application |
|---|---|---|
| 0 | 163.1 | 1 (163.1/163.1) |
| 1 | 32.99 | 0.20 |
| 2 | 17.47 | 0.11 |
| 3 | 11.65 | 0.07 |
| 4 | 8.91 | 0.05 |
| 5 | 7.43 | 0.05 |
| 6 | 6.34 | 0.04 |
| 7 | 5.68 | 0.03 |
| 8 | 5.13 | 0.03 |
| 9 | 4.76 | 0.03 |
| 10 | 4.36 | 0.03 |
| 12 | 3.65 | 0.02 |
| 14 | 3.35 | 0.02 |
| 16 | 3.1 | 0.02 |

From the results in Table 3 above, it was confirmed that when an ultrasonic wave was applied to the aqueous solution containing the high-molecular-weight hyaluronic acid of Example 5, the precursor, the viscosity decreased as the time for applying the ultrasonic wave increased. In addition, it was confirmed that the viscosity decreased rapidly within 1 to 2 seconds when the ultrasonic wave began to be applied, and the viscosity converged to about 3 cP after 10 minutes.

**Experimental Example 3: Measurement of molecular weight change before and after ultrasonic wave application**

Hyaluronic acid sodium salt (0.02 g) with a molecular weight of $1.2 \times 10^6$ g/mol was placed in a mass cylinder, and 0.9% (w/v) NaCl was added and dissolved to make a total volume of 20 mL to prepare an aqueous solution of hyaluronic acid sodium salt. 0.8 mL of the aqueous solution was taken, 0.1 M $Na_2SO_4$ was added, and the solution was prepared so that the total volume was 10 mL. Thereafter, distilled water was added to the solution to dilute the concentration by 2 times, thereby preparing a High-Performance Liquid Chromatography (HPLC) analysis sample.

The solution prepared in the same manner as in Example 5 was charged to the ultrasonic wave generator, and an ultrasonic wave was applied to prepare an aqueous solution containing a high-molecular-weight hyaluronic acid structure. A sample for Gel Permeation Chromatography (GPC) measurement was prepared using the aqueous solution prepared above.

To analyze the concentration and molecular weight of the HPLC analysis sample and the GPC measurement sample, HPLC (e2695, waters) and GPC column (OHPak SB-802.5 HQ 8.0 x 300mm, 6um, Shodex) were used. A 0.1 M

sodium sulfate (Na$_2$SO$_4$) aqueous solution prepared by dissolving 14.2 g of sodium sulfate anhydrous (DAEJUNG) in 1 L of purified water was used as the mobile phase. The column temperature was maintained at 40°C and the oven temperature was maintained at 25°C, 100% 0.1 M sodium sulfate (Na$_2$SO$_4$) was added, and the sample was stabilized in the oven for 1 hour at a flow rate of 1.0 mL/min. Afterwards, the sample was filtered with a syringe filter (PVDF, 0.2 um, $\Phi$ 13 mm), and then 20 $\mu$L was injected into the GPC column and analyzed at a UV wavelength of 210 nm for 16 minutes.

In FIG. 6, (a) shows the molecular weight of hyaluronic acid sodium salt, which is the precursor of the structure according to the present invention, and (b) shows the molecular weight of the high-molecular-weight hyaluronic acid structure according to the present invention.

It was confirmed that the molecular weight of hyaluronic acid, the precursor of the structure according to the present invention, and the molecular weight of the high-molecular-weight hyaluronic acid structure according to the present invention were the same with almost no change.

**Experimental Example 4: Preparation of viscosity-controlled solution**

Using an ultrasonic wave homogenizer, an ultrasonic wave was applied to the aqueous solution of hyaluronic acid sodium salt, the precursor, prepared in Example 5 at 25°C for about 16 minutes to prepare an aqueous solution (a) containing a high-molecular-weight hyaluronic acid structure. In addition, an aqueous solution of hyaluronic acid sodium salt (b) to which ultrasonic waves were not applied was prepared, and a solution mixed with the aqueous solution (a) was prepared.

The viscosity of the mixed solution of the aqueous solution (a) and the aqueous solution (b) was compared and shown in Table 4 below.

[Table 4]

| Proportion of aqueous solution (a) (%) | Proportion of aqueous solution (b) (%) | viscosity(cp) |
|:---:|:---:|:---:|
| 100 | 0 | 2.5 |
| 99 | 1 | 3.2 |
| 96 | 4 | 3.7 |
| 90 | 10 | 5.3 |
| 0 | 100 | 165 |

From the results in Table 4 above, it was found that when the aqueous solution (a) containing the high-molecular-weight hyaluronic acid structure according to the present invention is mixed with about 10% of the aqueous solution (b) to which ultrasonic waves are not applied, a solution with a viscosity value of 3 to 6 cP can be prepared.

**Experimental Example 5: Preparation of viscosity-controlled ophthalmic composition**

Hyaluronic acid sodium salt (sodium hyaluronate, a product from Shiseido company, Japan) (0.3 g) with a molecular weight of 1.0 x 10$^6$ g/mol was placed in a mass cylinder, and distilled water was added so that the total volume was 100 ml. At 25°C, the solution was stirred until it became uniform, thereby preparing an aqueous solution of hyaluronic acid sodium salt with a concentration of 0.3% (w/v).

The prepared aqueous solution of hyaluronic acid sodium salt with a concentration of 0.3 (w/v)% was treated in the same manner as in Examples 1 to 5 to prepare an aqueous solution containing the high-molecular-weight hyaluronic acid structure.

By adding additives to the aqueous solution containing the high-molecular-weight hyaluronic acid structure prepared above, an ophthalmic composition comprising an aqueous solution containing the high-molecular-weight hyaluronic acid structure was prepared as shown in Table 5 below. The viscosity and osmotic pressure of the prepared ophthalmic composition were measured and listed in Table 5 below.

[Table 5]

|  |  | Ex. 6 | Ex. 7 | Ex. 8 | Ex. 9 | Ex. 10 | Ex. 11 |
|---|---|---|---|---|---|---|---|
| Main ingredient | Molecular weight (g/mol) | $1.0 \times 10^6$ | $1.0 \times 10^6$ | $1.0 \times 10^6$ | $1.0 \times 10^6$ | $1.0 \times 10^6$ | $1.0 \times 10^6$ |
|  | Conc. of hyaluronic acid sodium salt | 0.30 | 0.30 | 0.30 | 0.30 | 0.30 | 0.3% |
|  | Viscosity of aqueous solutions containing structure of polymer HA (cP) | 59 | 54 | 50 | 43 | 8.3 | 8.6 |
| Excipients | PBS (1×) | 10% | - | - | - | - | - |
|  | NaCl | - | 0.80% | 0.68% | 0.70% | - | - |
|  | Additives | - | - | - | - | 0.31% | 0.29% |
| Viscosity after adding excipients |  | 24.1 | 27.3 | 22.0 | 24.7 | 2.5 | 2.7 |
| Osmotic pressure (mOsmol/kg) |  | 319 | 268 | 275 | 300 | 276 | 284 |

In Table 5, one or more of phosphate-buffered saline (PBS), sodium chloride (NaCl), and additives were used as excipients. The additive was a mixture containing 0.01% of ethylenediaminetetraacetic acid disodium salt (EDTA-2Na), 0.19% of aminocaproic acid, 0.69% of sodium chloride (NaCl), 0.15% of potassium chloride (KCl), and distilled water.

From the results in Table 5 above, it was confirmed that by adding various excipients to the aqueous solution containing the high-molecular-weight hyaluronic acid structure according to the present invention, it was possible to provide an ophthalmic composition with a viscosity in the range of 1 to 30 cP.

**Experimental Example 6: Evaluation of water retention capacity of aqueous solutions in Examples 1, 3 and 5 and Comparative Example 1**

The water retention capacity of the aqueous solutions in Examples 1, 3 and 5, and Comparative Example 1 was measured and evaluated.

1 g of each sample was collected, placed in a 10cc vial, left at 25°C for 1 hour, and the weight change was measured. The results are shown in Table 6 below.

[Table 6]

|  | Comparative Example 1 | Example 1 | Example 3 | Example 5 |
|---|---|---|---|---|
| **Weight reduction ratio (%)** | 8.85 | 7.8 | 7.6 | 7.5 |

From the results in Table 6 above, it was confirmed that although the viscosity of the aqueous solution of hyaluronic acid sodium salt treated using a high pressure disperser or ultrasonic wave homogenizer was lower than that of the pure aqueous solution of hyaluronic acid sodium salt (Comparative Example 1), the weight change was small. Through this, it was found that the aqueous solution containing the structure of hyaluronic acid according to the present invention had low viscosity and excellent moisture retention capacity. It could be found that this result is suitable for use in artificial tears, etc., which are necessary to improve moisture retention capacity.

**Experimental Example 7: Contact angle measurements of aqueous solutions in Examples 1, 3 and 5 and Comparative Example 1**

The contact angle was measured for the aqueous solutions prepared in Examples 1, 3 and 5, and Comparative Example 1 above. Contact angles were measured on hydrophilic and hydrophobic glass surfaces, respectively. The measured contact angles are listed in Table 7 below.

[Table 7]

|  | Comparative Example 1 | Example 1 | Example 3 | Example 5 |
|---|---|---|---|---|
| **Contact angle on hydrophilic glass surface (°)** | 19 | 25 | 26 | 27 |
| **Contact angle on hydrophobic glass surface (°)** | 106 | 98 | 96 | 95 |

From the results in Table 7, on the hydrophilic glass surface, the contact angle of the aqueous solution prepared in Examples 1, 3 and 5 is greater than Comparative Example 1, and on the hydrophobic glass surface, the contact angle of the aqueous solution prepared in Examples 1, 3 and 5 is smaller than Comparative Example 1. Therefore, it was found that the aqueous solutions prepared in Examples 1, 3 and 5 were relatively lipophilic.

**Experimental Example 8: Intrinsic viscosity test**

When the reduced viscosity is calculated by measuring the viscosity of the aqueous solution to be measured according to the concentration and then extrapolated, the reduced viscosity value at concentration of 0 is called intrinsic viscosity. As mentioned above, intrinsic viscosity refers to the volume per unit mass of a polymer when there is no interaction between polymer chains in the solvent.

Using distilled water, the prepared aqueous solution of hyaluronic acid sodium salt 0.5(w/v)% was diluted with distilled water to the concentration of 0.3(w/v)%, 0.1(w/v)%, 0.05(w/v)%, 0.01(w/v)%, and 0.005(w/v)%, respectively. The viscosity at 25°C was measured using a Brookfield DV2T viscometer, calculated as reduced viscosity, and extrapolated to obtain intrinsic viscosity. Hyaluronic acid sodium salt with a molecular weight of 1.2 million has an intrinsic viscosity of 2.3 $m^3$/kg, and hyaluronic acid sodium salt with a molecular weight of 900,000 has an intrinsic viscosity of 1.8 $m^3$/kg.

The aqueous solution containing the hyaluronic acid structure with a molecular weight of 900,000 prepared in Example 5 above was calculated to have an intrinsic viscosity of about 0.8 $m^3$/kg.

**Claims**

1. A high-molecular-weight hyaluronic acid structure, which is derived from high-molecular-weight hyaluronic acid, wherein the viscosity of solution containing the structure is 1 to 60 cP.

2. The high-molecular-weight hyaluronic acid structure according to claim 1, wherein the molecular weight of the hyaluronic acid is in the range of $5.0 \times 10^5$ to $2.0 \times 10^6$ g/mol.

3. The high-molecular-weight hyaluronic acid structure according to claim 1, wherein solution containing the structure contains hyaluronic acid at 0.01 % (w/v) to 0.5 % (w/v).

4. The high-molecular-weight hyaluronic acid structure according to claim 1, wherein when the molecular weight and concentration of the structure and hyaluronic acid, a precursor of the structure, are the same, a/b, which is the ratio of the viscosity (a) of solution containing the structure and the viscosity (b) of solution containing hyaluronic acid, a precursor of the structure, is 0.001 to 0.30.

5. The high-molecular-weight hyaluronic acid structure according to claim 1, wherein the intrinsic viscosity of solution containing the structure is 0.1 to 2.5 $m^3$/kg.

6. An ophthalmic composition, comprising the high-molecular-weight hyaluronic acid structure according to claim 1.

7. The ophthalmic composition according to claim 6, wherein the viscosity of the ophthalmic composition is 1.0 to 30.0 cP.

8. The ophthalmic composition according to claim 6, further comprising excipients.

9. The ophthalmic composition according to claim 8, wherein the excipients are at least one selected from the group consisting of buffering agents, preservatives, tonicity regulators, and pH regulators.

10. The ophthalmic composition according to any one of claims 6 to 9, which is for the prevention or treatment of dry eye syndrome.

11. A kit, comprising a container containing the ophthalmic composition according to any one of claims 6 to 9, wherein the container includes a dispensing means suitable for topical administration of the ophthalmic composition.

12. The kit according to claim 11, wherein the dispensing means provides the ophthalmic composition dropwise in droplets having a volume of 0.01 to 0.10 ml.

【Figure 1】

【Figure 2】

【Figure 3】

【Figure 4】

【Figure 5】

【Figure 6】

## INTERNATIONAL SEARCH REPORT

| International application No. |
| --- |
| **PCT/KR2023/002353** |

### A. CLASSIFICATION OF SUBJECT MATTER

**C08B 37/08**(2006.01)i; **A61K 9/00**(2006.01)i; **A61K 47/36**(2006.01)i; **A61P 27/02**(2006.01)i; **A61P 27/04**(2006.01)i; **A61K 9/08**(2006.01)i

According to International Patent Classification (IPC) or to both national classification and IPC

### B. FIELDS SEARCHED

Minimum documentation searched (classification system followed by classification symbols)

C08B 37/08(2006.01); A61K 47/10(2006.01); A61K 47/18(2006.01); A61K 47/26(2006.01); A61K 47/36(2006.01); A61K 9/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루론산(hyaluronic acid), 점안액(eye drop), 저점도(low viscosity), 전단응력 (shear stress)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2018-0014825 A (PRESBYOPIA THERAPIES, LLC) 09 February 2018 (2018-02-09)<br>See claims 9-16; and paragraphs [0072]-[0103]. | 1,3-9,11-12 |
| Y | | 2,10 |
| Y | KR 10-2014-0041586 A (SANTEN PHARMACEUTICAL CO., LTD.) 04 April 2014 (2014-04-04)<br>See paragraphs [0030]-[0034]. | 2,10 |
| Y | KR 10-2268002 B1 (HUONS CO., LTD.) 23 June 2021 (2021-06-23)<br>See claim 1; paragraphs [0018]-[0019], [0024], [0026], [0037] and [0051]-[0053]; and table 1. | 1-12 |
| Y | JP 2019-518789 A (BOSTON SCIENTIFIC SCIMED, INC.) 04 July 2019 (2019-07-04)<br>See paragraphs [0010] and [0028]. | 1-12 |

☑ Further documents are listed in the continuation of Box C.  ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **15 May 2023** | **15 May 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/002353**

**C. DOCUMENTS CONSIDERED TO BE RELEVANT**

| Category* | Citation of document, with indication, where appropriate, of the relevant passages | Relevant to claim No. |
|---|---|---|
| Y | KR 10-2020-0085215 A (HUONS CO., LTD.) 14 July 2020 (2020-07-14)<br>See claims 1 and 3-4; and paragraph [0004]. | 1-12 |

Form PCT/ISA/210 (second sheet) (July 2022)

**INTERNATIONAL SEARCH REPORT**
Information on patent family members

International application No.

**PCT/KR2023/002353**

| Patent document cited in search report | | | Publication date (day/month/year) | Patent family member(s) | | | Publication date (day/month/year) |
|---|---|---|---|---|---|---|---|
| KR | 10-2018-0014825 | A | 09 February 2018 | CN | 107847492 | A | 27 March 2018 |
| | | | | CN | 107847492 | B | 25 May 2021 |
| | | | | CN | 107920984 | A | 17 April 2018 |
| | | | | EP | 3310336 | A1 | 25 April 2018 |
| | | | | EP | 3310336 | A4 | 05 December 2018 |
| | | | | EP | 3310356 | A1 | 25 April 2018 |
| | | | | EP | 3310356 | A4 | 05 December 2018 |
| | | | | JP | 2018-517738 | A | 05 July 2018 |
| | | | | JP | 2018-517740 | A | 05 July 2018 |
| | | | | JP | 2021-035954 | A | 04 March 2021 |
| | | | | JP | 6835747 | B2 | 24 February 2021 |
| | | | | JP | 6838712 | B2 | 03 March 2021 |
| | | | | JP | 7026189 | B2 | 25 February 2022 |
| | | | | KR | 10-2018-0014824 | A | 09 February 2018 |
| | | | | US | 2015-0065511 | A1 | 05 March 2015 |
| | | | | US | 9089562 | B2 | 28 July 2015 |
| | | | | WO | 2016-205068 | A1 | 22 December 2016 |
| | | | | WO | 2016-205069 | A1 | 22 December 2016 |
| KR | 10-2014-0041586 | A | 04 April 2014 | CN | 103608000 | A | 26 February 2014 |
| | | | | CN | 103608000 | B | 11 May 2016 |
| | | | | EP | 2684560 | A1 | 15 January 2014 |
| | | | | EP | 2684560 | A4 | 06 August 2014 |
| | | | | JP | 2013-028599 | A | 07 February 2013 |
| | | | | JP | 5981783 | B2 | 31 August 2016 |
| | | | | KR | 10-1906631 | B1 | 10 October 2018 |
| | | | | US | 2014-0088039 | A1 | 27 March 2014 |
| | | | | WO | 2012-176865 | A1 | 27 December 2012 |
| KR | 10-2268002 | B1 | 23 June 2021 | KR | 10-2020-0080493 | A | 07 July 2020 |
| JP | 2019-518789 | A | 04 July 2019 | CN | 109789219 | A | 21 May 2019 |
| | | | | CN | 113730605 | A | 03 December 2021 |
| | | | | EP | 3442589 | A1 | 20 February 2019 |
| | | | | JP | 2020-189215 | A | 26 November 2020 |
| | | | | JP | 2022-164780 | A | 27 October 2022 |
| | | | | JP | 6757464 | B2 | 16 September 2020 |
| | | | | JP | 7134202 | B2 | 09 September 2022 |
| | | | | US | 2018-0021252 | A1 | 25 January 2018 |
| | | | | WO | 2018-017861 | A1 | 25 January 2018 |
| KR | 10-2020-0085215 | A | 14 July 2020 | KR | 10-2051356 | B1 | 03 December 2019 |
| | | | | KR | 10-2481389 | B1 | 28 December 2022 |
| | | | | WO | 2020-141711 | A1 | 09 July 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220023811

- KR 1020210015377